# EUROPEAN PATENT APPLICATION

(11) **EP 4 678 738 A1**
(43) Date of publication of application: **14.01.2026**
(21) Application number: 24306178.5
(22) Date of filing: 12.07.2024
(51) Int. Cl.: C12N 5/077

(54) **METHOD FOR GRADING THEN POOLING MESENCHYMAL STROMAL CELLS**

(71) Applicant: Assistance Publique - Hôpitaux de Paris, 75012 Paris (FR); Université Paris Cité, 75006 Paris (FR)
(72) Inventor: MEBARKI, Miryam, 75010 PARIS (FR); CRAS, Audrey, 75010 PARIS (FR)
(74) Representative: Flesselles, Bruno F.G.

(57) **Abstract**

The invention pertains to methods for preparing pools of mesenchymal stromal cells for treatment of immune or inflammatory diseases, by screening cells from individual donors and including cells with high immunomodulatory properties in the pools.

## Description

The invention pertains to methods for preparing Mesenchymal stromal cells (MSCs) for treatment of immune or inflammatory diseases.

Mesenchymal stromal cells (MSCs) have been extensively studied for the development of advanced therapy medicinal products (ATMPs), in particular cell and tissue engineered therapies. However, to date, only a few MSC-based cellular therapies have been approved, highlighting the complexity to transfer MSC-based products from the bench to the market. The main challenge is the high heterogeneity of MSC biological properties, which makes it difficult to standardize production processes and predict the clinical response. This variability can be related to culture conditions, be tissue-specific or donor-dependent (François et al., Mol Ther. 2012 Jan;20(1): 187-95.). It is known that umbilical cord-derived MSCs (UC-MSCs) display higher immunoregulatory properties than MSCs derived from adult tissues (bone marrow, adipose tissue), and therefore are favored as more interesting for the treatment of immune and/or inflammatory diseases (Mebarki et al. Stem Cell Res Ther. 2021 Feb 26;12(1):152). However, the donor-to-donor variability of UC-MSCs is more important compared to adult tissues derived MSCs (Wang Z, Chai C, Wang R, Feng Y, Huang L, Zhang Y, et al. Single-cell transcriptome atlas of human mesenchymal stem cells exploring cellular heterogeneity. Clin Transl Med. 2021 Dec 29;11(12):e650).

Previous study performed on UC-MSCs isolated from 12 donors emphasized the variable yield of cell isolation and of their proliferative capacities (Mebarki et al., Stem Cell Res Ther. 2021 Nov 13;12(1):571). The lack of predictive biomarkers to identify optimal donors makes it difficult to predict the therapeutic effect. Currently, the majority of products in development use MSCs isolated from a single donor, with the risk of an insufficient clinical response if used cells have poor biological properties. Several strategies have been tested to enhance MSC properties and reduce the donor-dependent heterogeneity, including hypoxic or cytokines preconditioning to normalize the immunomodulatory functions of UC-MSCs. However, transcriptomic analysis of MSCs revealed the persistence of variations in biological properties after cytokines treatment and was associated with a decrease of cell cycle division. Hypoxic conditions allowed to preserve proliferative capacities of umbilical cord blood-derived MSCs without standardizing their functions between donors, underlying the heterogeneity of MSCs response to hypoxia.

More recently, pooling MSCs isolated from multiple donors has emerged as a new strategy to reduce the donor-dependent heterogeneity. Few protocols have been tested in clinic, including graft-versus-host disease and critical limb ischemia, using mostly bone marrow-derived MSCs. They suggested the clinical benefit of pooled MSCs from multiple donors compared with MSCs isolated from a single donor. Conversely, Hejretova et al. (Cell Tissue Bank. 2020 Mar;21(1):119-129) showed similar immunomodulatory properties between single donors and pooled MSCs. These differences may be explained by the strategy of current protocols, which aim to pool MSCs from randomly donors. A prior selection of donors, could improve biological properties of pooled MSCs. For example, UC-MSCs pooled based on the gender of born donors, has showed differences in proliferation and cytokines expression (Kannan et al, Stem Cell Rev and Rep. 2022;18(5):1851-64.

The present invention provides a method for grading the immunomodulatory property of MSCs, which makes it possible to select the donors whose cells are to be included in pools. The inventors indeed assessed human UC-MSCs derived from 10 donors and were able to confirm variations in their biological properties. Based on the immunoregulatory functions of UC-MSCs in vitro, the inventors propose to grade or rank the profiles of donors as "high potential" or "not high potential" to make sure that cells from a "high potential" donor can be included in a pool with cells of "not high potential" donor(s). The inventors were also able to grade the cells in three profiles (the "not high potential" include "medium potential" or "low potential" cells). Using MSCs with a high immunomodulatory potential or property makes it possible to increase the therapeutic potential (immune properties) of the pooled cells even though the pool contains cells with a lower potential.

Using the cells of one "high" donor in various pools makes it possible to increase the number of batches of ATMPs, and hence to treat more patients, with a higher expectation of therapeutic success. This is of interest, as only a handful of donors present this "high" potential property with regards to the cell's immunomodulatory property.

In 2006, the International Society for Cellular Therapy (ISCT) established the following unified and minimal criteria to define MSCs (Dominici et al,. Int Soc Cell Ther position statement Cytotherapy. 2006;8(4):315-7).
- Plastic-adherent cells
- Expression of the surface markers CD73, CD90 and CD105, but not the hematopoietic markers CD45, CD34, CD14, CD11b, CD19, CD79a or HLA-DR
- Trilineage mesenchymal differentiation capacity into osteoblasts, adipocytes and chondrocytes.

It is reminded that the immunomodulatory properties of mesenchymal stromal/stem cells (MSCs) are a key aspect of their therapeutic potential. Their immunomodulatory characteristics include
- Immunosuppression: They can inhibit naive and memory T-cell responses and communicate with antigen-presenting cells, thereby modulating immune responses.
- Regulation of Immune and Inflammatory Responses: they secrete cytokines, chemokines, signaling molecules, and growth factors, which effectively contribute to the regulation of immune and inflammatory responses.
- Modulation of Immune Cells: they can interact with innate and adaptive immune systems via cell-to-cell interactions and immunomodulatory/regenerative factors, affecting the phenotype and functional properties of monocytes/macrophages, dendritic cells, T cells, and B cells.

In summary, in the view of their immunomodulatory properties, MSCs have a therapeutic potential for the treatment of a variety of immune-mediated diseases, including multiple sclerosis (MS), osteoarthritis, rheumatoid arthritis (RA), collagen-induced arthritis, Type 1 diabetes mellitus, systemic lupus erythematosus, inflammatory bowel diseases (including Crohn's disease and ulcerative colitis), inflammatory airway & pulmonary diseases (such as obstructive lung diseases (COPD), asthma, idiopathic pulmonary fibrosis (IPF), acute respiratory distress syndrome (ARDS), Bronchial pulmonary dysplasia), graft versus host disease (GvHD), and allergic disorders. They can also be used in the treatment of infectious diseases (Sharma et al, World J Stem Cells. 2021 Jun 26; 13(6): 568-593). One can also cite the diseases mentioned in Hoang et al (Signal Transduct Target Ther. 2022; 7: 272), who cites cardiovascular diseases (see also Hare, J.M. et al. J. Am. Med. Assoc. 308, 2369-2379 (2012)), digestive system diseases (see also above), liver diseases (liver failure, cirrhosis, cancer, alcoholic liver disease, nonalcoholic fatty liver disease (NAFLD), and autoimmune liver disease (ALD)), and indicates that bone marrow MSCs are good candidates for brain and spinal cord injury treatment. Adipose Tissue MSCs (AT-MSCs) are good candidates for reproductive disorder treatment and skin regeneration, and UC-MSCs are good candidates for pulmonary disease and acute respiratory distress syndrome treatment.

The invention thus relates to a method for evaluating and grading the immunomodulatory property of a population of human mesenchymal stromal cells (MSCs), comprising
a. Revealing immunomodulatory properties of mesenchymal stromal cells cultured in a resting state and/or primed in pro-inflammatory conditions
b. Obtaining a numerical value associated with the immunomodulatory property of the cells
c. Grading the immunomodulatory property of the population of cells as high, medium or low, wherein a grade "high" or "medium" is assigned when the immunomodulatory property of the population of the cells is above the median, and wherein a grade "low" is assigned when the immunomodulatory property of the population of the cells is below the median, wherein the median has been obtained from a series of numerical values associated with the immunomodulatory properties of multiple populations of mesenchymal stromal cells from multiple subjects.

This method thus makes it possible to select donors the cells of whom will be included in pools, and in particular to identify donors the cells of whom have a high immunomodulatory potential and ability. Such method is performed on an aliquot of MSCs isolated from a donor then cultured *in vitro.*

Thus pools of MSCs can be prepared with prior selection of donors (and thus not randomly), in order to obtain a composition where the functions of cells whose immunomodulatory properties are low are improved. The donors whose cells have high immunomodulatory properties are qualified "high" donors.

The MSCs are first cultured either in a resting state or in pro-inflammatory conditions. In particular, the cells may be cultured in the presence of pro-inflammatory cytokines. Pro-inflammatory cytokines are signaling molecules that are involved in the inflammatory response. They are produced by various cells, notably immune cells, and help to regulate the immune response, promote inflammation, and recruit immune cells to the site of infection or injury. As pro-inflammatory cytokines, one can cite Tumor Necrosis Factor-alpha (TNF-α), Interleukin-1 (IL-1) (notably IL-1α or IL-1β), Interleukin-6 (IL-6), Interleukin-8 (IL-8), Interleukin-12 (IL-12), Interleukin-17 (IL-17), Interferon-gamma (IFN-y), or Granulocyte-Macrophage Colony-Stimulating Factor (GM-CSF).

Culture in the presence of the pro-inflammatory cytokines "primes" the MSCs thereby initiating, enhancing or modifying their response to subsequent stimuli. In particular, such priming would make the cells able to interact with cells of the immune system. Immunomodulation ability of the cells can thus be determined. Such response can increase cytokine production (such as IL-6, IL-8, IL-10 (immunosuppressive effect), various Growth Factors, Chemokines, and Signaling Molecules (in particular VEGF that plays a crucial role in cell engraftment, neovascularization, and wound healing), Intercellular adhesion molecule-1 (ICAM-1) and vascular cell adhesion molecule 1 (VCAM-1) or IDO (Indoleamine 2,3-dioxygenase), the presence of which being associated with the suppression of inflammatory processes).

The immunomodulating property of the primed MSCs can thus be evaluated in any assay that is known in the art. One can cite:
- Cytokine Production Assays such as ELISA (Enzyme-Linked Immunosorbent Assay) for measuring amount of a give cytokine, or of multiple cytokines, in Multiplex Cytokine Assays,
- Flow Cytometry, to identify and quantify expression of surface markers indicative of activation, differentiation, or specific cell subsets, or of intracellular cytokine (Intracellular Cytokine Staining),
- Phagocytosis and Killing Assays, to evaluate phagocytic activity, or ability to kill pathogens,
- Proliferation Assays of immune or inflammatory cells, such as BrdU/EdU Incorporation Assay, using thymidine analogs, CFSE Dilution Assay, using CFSE (a fluorescent dye) and analysis by flow cytometry,
- Gene Expression Analysis such as qPCR (Quantitative Polymerase Chain Reaction), RNA-Sequencing,
- Functional Assays, such as Mixed Lymphocyte Reaction (MLR), where the primed or non-primed cells are co-cultured with allogeneic T-cells, and T-cell proliferation is measured, or Cytotoxicity Assays, to measure the ability of the primed cells to kill target cells.

In one embodiment, the immunomodulatory property of the cells is assessed by the expression of markers, notably by ELISA, RNA-sequencing or flow cytometry. In this embodiment, it is preferred to assess expression of at least one marker selected from IDO, Inter Cellular Adhesion Molecule-1 (ICAM-1/CD54), programmed death ligand 1 (PD-L1/CD274), vascular cell adhesion molecule 1 (VCAM-1/CD106), CD200. Assessment of production of IDO, ICAM-1 or VCAM-1 is preferred. Amount of the molecule is measured before (basal or resting state) and after (primed state) culture in presence of the pro-inflammatory cytokines.

In another embodiment, the immunomodulatory property of the cells is assessed by Mixed lymphocyte reaction (MLR). MLR assay can be performed on MSCs both in a resting state (NT) or after priming for 48 h with IFNγ, TNFα and IFNγ + TNFα. Briefly, peripheral blood mononuclear cells (PBMCs) pooled from 10 allogenic donors can be co-cultured with MSCs at various (0:1 (control), 1:5, 1:10, 1:30, 1:100, 1:300 and 1:1000) MSCs:PBMC ratio with a constant amount of PBMC (3 × 10⁵) and a decreasing amount of UC-MSCs from 6 × 10⁴ down to 3 × 10². PBMCs are then labeled with labeled antibodies against CD3, CD45 and 7-AAD and analyzed by flow cytometry. It is reminded that MSCs have immunomodulatory properties and would thus inhibit proliferation of the lymphocytes in the PBMCs (as compared to proliferation observed with the control).

In particular, Mixed Lymphocyte Reaction assay is performed with MSCs in a resting state, at a 1:30 MSCs:PBMC ratio (using 3 × 10⁵ PBMCs).

Any above method makes it possible to obtain a numerical value. When performing MLR, the numerical value may be the percentage of inhibition of lymphocyte proliferation in presence of MSCs (as compared to the proliferation in the absence of MSCs). When measuring expression of a marker, the numerical value may be the percentage of increase or decrease of expression of the markers (as compared to the expression measured in cells in the native state).

In order to grade or classify the immunomodulatory properties of the cells of a given donor, the numerical value obtained is compared to a set of values that has been obtained from prior analysis of a series of MSCs from multiple donors. It is advisable to use a series of 10 or more donors, in order to obtain a diversity of donors, so that the used donors include donors with cells of high immunomodulatory properties.

From the different values obtained from the analysis of the immunomodulatory properties of the MSCs from multiple donors, it is possible to determine a median value, and quartile (first quartile, third quartile) values. Such median and quartile values are used in the method herein disclosed.

When the numerical value obtained for the tested MSCs is below the median value, a grade "low" is assigned to the cells. This indicates that the MSCs of the donor have low immunomodulatory ability. Such cells are not to be used alone, but may still be included in a pool with cells with a higher grade.

When the numerical value for the tested MSCs is above the median, the cells are graded "medium" or "high". This indicates that the cells could be used as such (alone) for treating a patient. However, it is preferred to use the cells in pools with cells of a "low" grade. This makes it possible to prepare multiple and large batches of ATMPs and increase the number of treated patients, with the pools of cells.

In another embodiment, the grade "high" is assigned when the numerical value associated with the immunomodulatory property of the cells is within the last quartile (quartile with the higher values), and wherein the grade "medium" is assigned when the numerical value associated with the immunomodulatory property of the cells is within the third quartile (between the median and the third quartile) of the series of numerical values associated with the immunomodulatory properties of the multiple populations of mesenchymal stromal cells from multiple donors.

This embodiment makes it possible to select the best ("high") cells from a donor to make sure that they are included in pools of MSCs used for preparing ATMPs.

In another embodiment, the grade "high" is assigned when the numerical value associated with the immunomodulatory property of the cells is above the sum of the median and the interquartile value, and wherein the grade "medium" is assigned when the immunomodulatory property of the population of the cells is above the median and below the sum of the median and the interquartile value. Using this method of grading the cells may sometime be more appropriate, providing three grades (high, medium and low) and giving more options for pooling the cells.

It is understood that the purpose is to classify the cells in comparison to each other. There may be some variability in the median and interquartile values when different series of MSCs from multiple donors. The examples provide an illustration of a value of the numbers that can be used when MLR is used as an assay for testing the immunomodulatory properties of the MSCs.

In the preferred embodiment, the mesenchymal stromal cells were isolated from human umbilical cord (umbilical cord-derived MSCs (UC-MSCs)). MSCs can be isolated from Wharton's jelly by enzymatic methods or explant methods.

In another embodiment, the mesenchymal stromal cells have been obtained from bone marrow. The mononuclear fraction is obtained through bone marrow aspiration, possibly followed by density gradient centrifugation. MSCs adhere to plastic and can be further separated from other cells in the mononuclear fraction.

In another embodiment, the mesenchymal stromal cells have been obtained from adipose tissue. Adipose tissue can be obtained through liposuction, and MSCs can be isolated by enzymatic methods or explant methods.

Using the grading system herein disclosed makes it possible to implement a method for preparing a composition of mesenchymal stromal cells, comprising:
a. Providing mesenchymal stromal cells from at least two donors
b. Separately testing the mesenchymal stromal cells of a., with regards to their immunomodulatory properties,
c. Selecting first mesenchymal stromal cells from a first donor, wherein the cells present high immunomodulatory properties
d. Selecting second mesenchymal stromal cells from a second donor, wherein the cells present medium or low immunomodulatory properties
e. Mixing the first and second selected mesenchymal stromal cells to obtain a pool of mesenchymal stromal cells.

This composition (or pools) of MSCs can be designated as "heterogenous", as it contains cells from different donors.

In b), the cells of each donor are separately tested, and can be graded according to their immunomodulatory properties, with grades being high, medium and low. This makes it possible to identify and select the donor the cells of whom have high immunomodulatory properties, and use the cells of this donor together with cells of another donor (potentially two other donors) who have cells of medium or low immunomodulatory properties.

MSCs from a "high" donor are mixed with MSCs from at least one "low" or "medium" donor.

When cells for one high donor are mixed with cells from one "medium" donor and cells from one "low" donor, the ratio may be 1:1:1 (same number of cells from each donors).

When cells for one high donor are mixed with cells from one "medium" donor, the ratio may be comprised between 1:5 and 1:1, preferably 1:2 and 1:1.

When cells for one high donor are mixed with cells from one "low" donor, the ratio may be comprised between 1:5 and 1:1, preferably 1:2 and 1:1.

Aliquots or bags of MSCs from each donor are amplified then can be either:
- individually frozen and pooled just before use.
- individually frozen and pooled just before the step of work cell stock.
- pooled and frozen just before the step of master cell stock.

Master cell stocks are prepared with the cells of each donor.

It is to be noted that, to date, it is generally not recommended to amplify MSCs more than a few times (about 4-5 passages) without the cells losing their properties (Mebarki et al, Stem Cell Research & Therapy, 2021). Hence, once a "high" donor has been identified, the clinical value of the cells is very high and it is preferred to advise to save them.

Using the cells of the pools for treatment of a patient would make it possible to obtain adequate clinical response, as shown in the examples. Indeed, the inventors showed that the presence of MSCs with high immunomodulatory properties, in a pool with other cells that do not have the same properties, makes it possible to obtain an adequate global immunomodulatory biological effect.

This would make it possible to observe a reduction in the variability of clinical responses, observed when MSCs which are derived from a single donor (consequently, each time the donor is changed, the response is different, making it difficult to anticipate), and to avoid the risk of inefficiency or low efficacy associated with the use of MSC derived from a single "low" donor or from a pool of several low and/or medium donors (the prior selection herein disclosed thus sets the method apart from prior art describing making pools, without prior donor selection).

In a preferred embodiment the testing and grading of the mesenchymal stromal cells is performed by the method disclosed above.

After mixing the cells of multiple donors, multiple aliquots of the cells of the pool can be prepared. The number of cells in each aliquot is such that between 10⁵ and 10⁷ cells/kg can be administered to a patient, using one aliquot or several aliquots.

The pool or the aliquots can be frozen -in DMSO 5 - 10% and then stored for cryopreservation in nitrogen at a temperature below -80°C, and generally at -120°C. It is advisable to prepare various master cell stocks (master banks) from each donor. Then an aliquot of the master cell stock from high donor and another from low and/or medium donor(s) may be thawed, cultured, expanded, then pooled just before freezing and cryopreservation to prepare the work cell stock. When there is a need of use, an aliquot of the work cell stock is thawed, the cells can be used for the treatment of one or more patients.

The invention thus also relates to a pool of mesenchymal stromal cells, wherein the pool contains cells from at least two donors, wherein the cells of one donor are graded as having high immunomodulatory property, and wherein the cells of the other donor(s) are graded as having medium or low immunomodulatory property.

The invention also relates to the pool as disclosed as a medicament or for use thereof as a medicament. One can also cite use of the pool as disclosed for the preparation of a medicament.

In particular the pool of mesenchymal stromal cells, wherein the pool contains cells from at least two donors, wherein the cells of one donor are graded as having high immunomodulatory property, and wherein the cells of the other donor(s) are graded as having medium or low immunomodulatory property, can be used for the treatment of immune or inflammatory diseases, or other diseases as indicated above.

A method for treating a patient in need thereof, comprising administering cells of a pool of mesenchymal stromal cells as herein disclosed to the patient can thus be implemented. In particular, the cells are provided at a dose comprised between 10⁵/kg to 10⁷/kg to the patient, in one or more administrations.

It is reminded that cells can be counted by any method known in the art: Malassez cell count, automated cell count with or without viability marker, flow cytometry with a specific marker.

As indicated above, it is possible to prepare a mesenchymal stromal cells for therapeutic use, by thawing a frozen pool or aliquot herein disclosed, and culturing and/or expanding the thawed cells. The cultured or expanded cells can then be used for one or more administration to a patient. The pools can also be used after thawing, without expanding cells.

### FIGURES

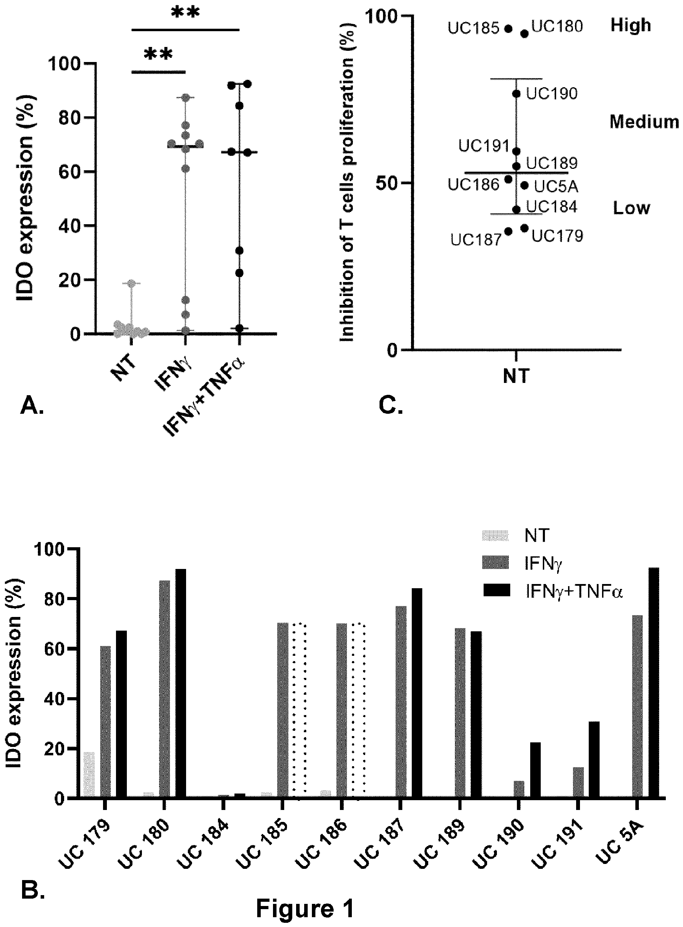

**Figure 1****: Description of immunomodulatory properties of each of the 10 donors. A.** Median (left graph) and individual (right graph) expression of IDO (%) at basal state (NT) and after pro-inflammatory priming by IFNγ and IFNγ + TNFα. UC 185 and UC 186 were not treated with IFNγ + TNFα and are presented in dotted lines. **B.** Inhibition of T-lymphocyte proliferation (%) assessed by MLR assay at basal state (NT) (ratio UC-MSCs:PBMC 1:30). n=1/donor.

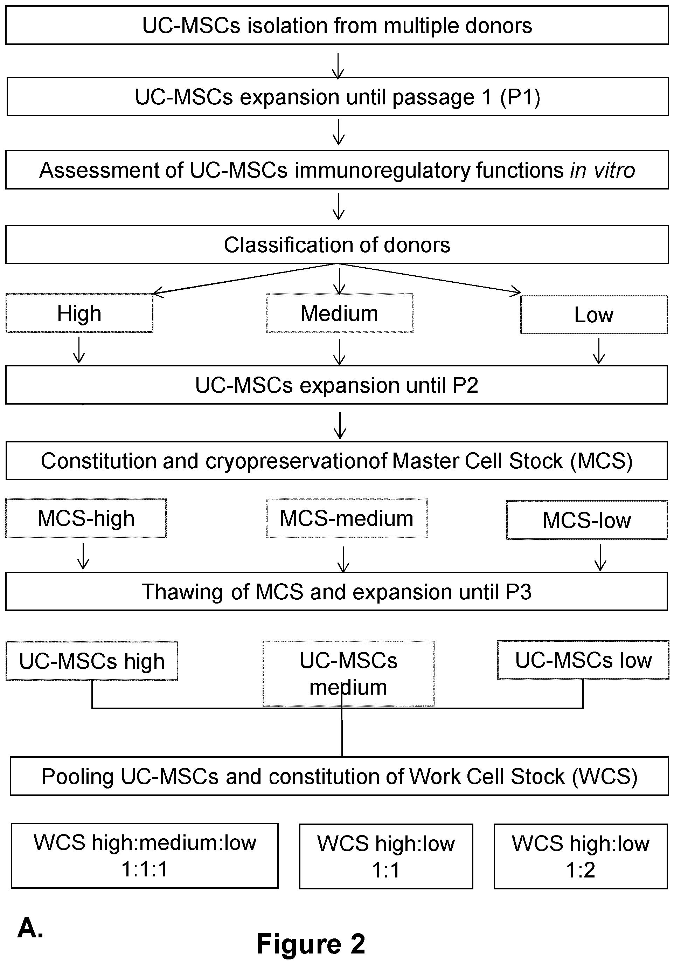

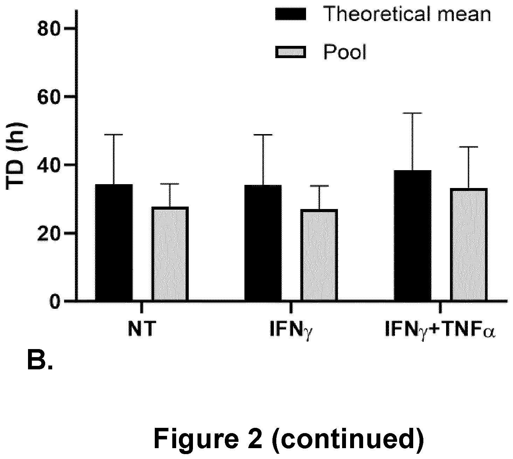

**Figure 2****: Constitution of UC-MSCs pools with prior selection of donors based on their immunoregulatory functions. A.** The process of constitution of UC-MSCs pools. Comparison of time doubling (TD) between **B.** the theoretical mean of individual donors and the pool.

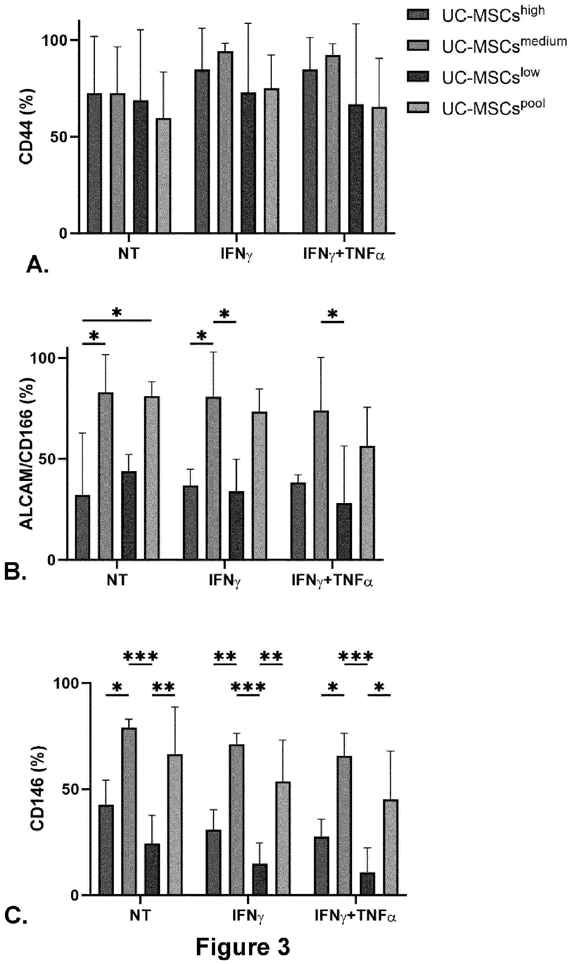

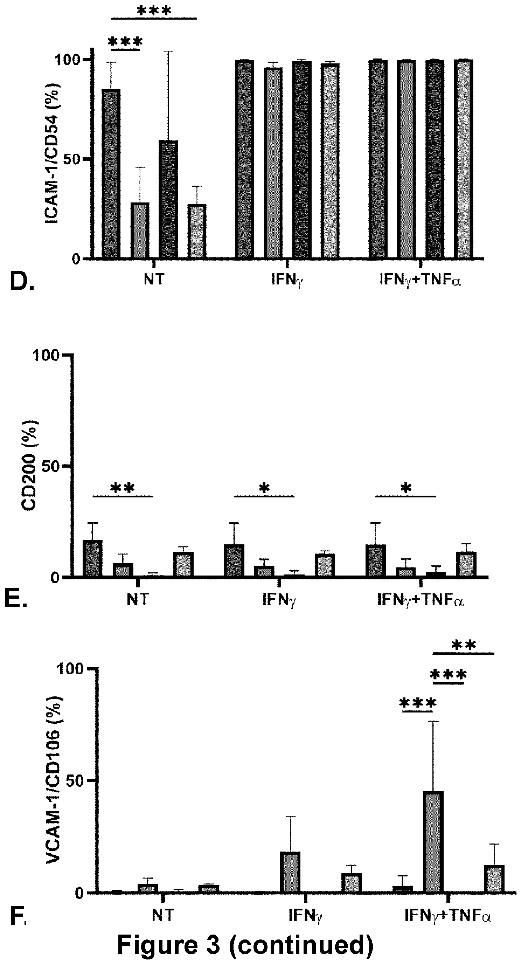

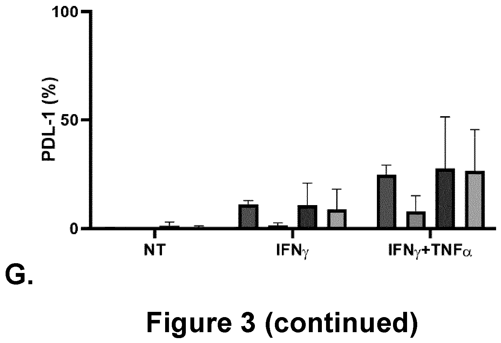

**Figure 3****: Expression of adhesion surface markers in UC-MSCs isolated from individual donors versus pool.** Expression (%) of **A.** CD44, **B.** ALCAM/CD166, **C.** CD146, **D.** ICAM-1/CD54, **E.** CD200, **F.** VCAM-1/CD106 and **G.** PDL-1 at basal state (non-treated) and after pro-inflammatory treatment with IFNγ and IFNγ+TNFα.

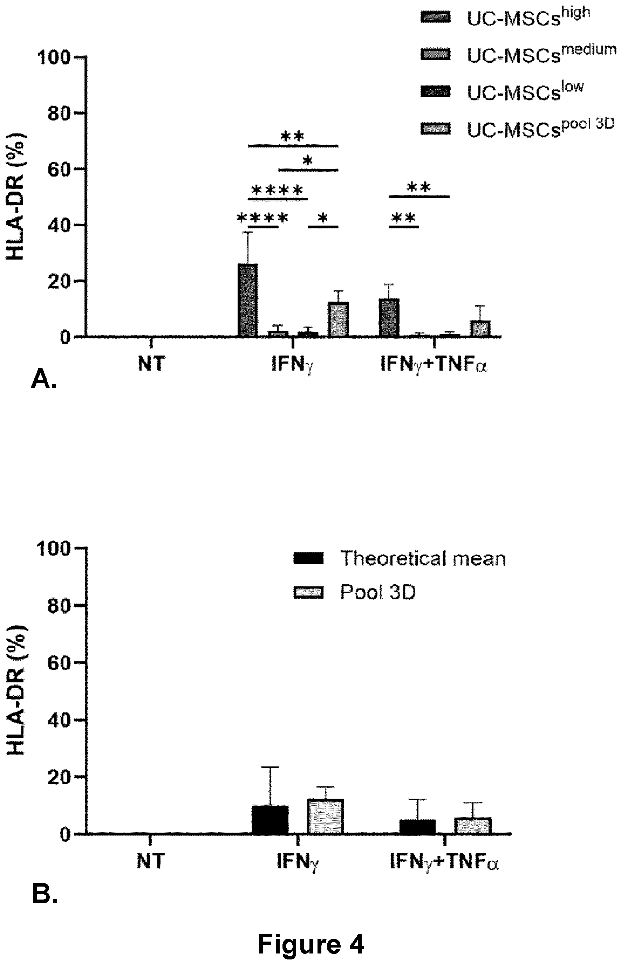

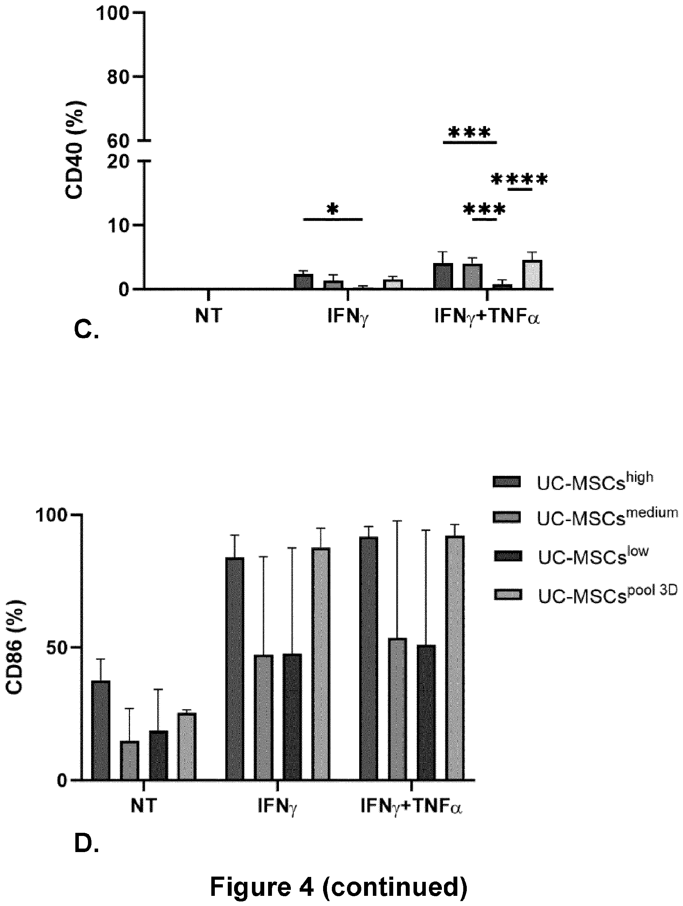

**Figure 4****: Expression of markers of immunogenicity in UC-MSCs isolated from individual donors versus pool.** Expression (%) of **A.** HLA-DR (individual donors versus pool), **B.** HLA-DR (theoretical mean of individual donors versus pool), **C.** CD86, **D.** CD40 at basal state (non-treated) and after pro-inflammatory treatment with IFNγ and IFNγ+TNFα.

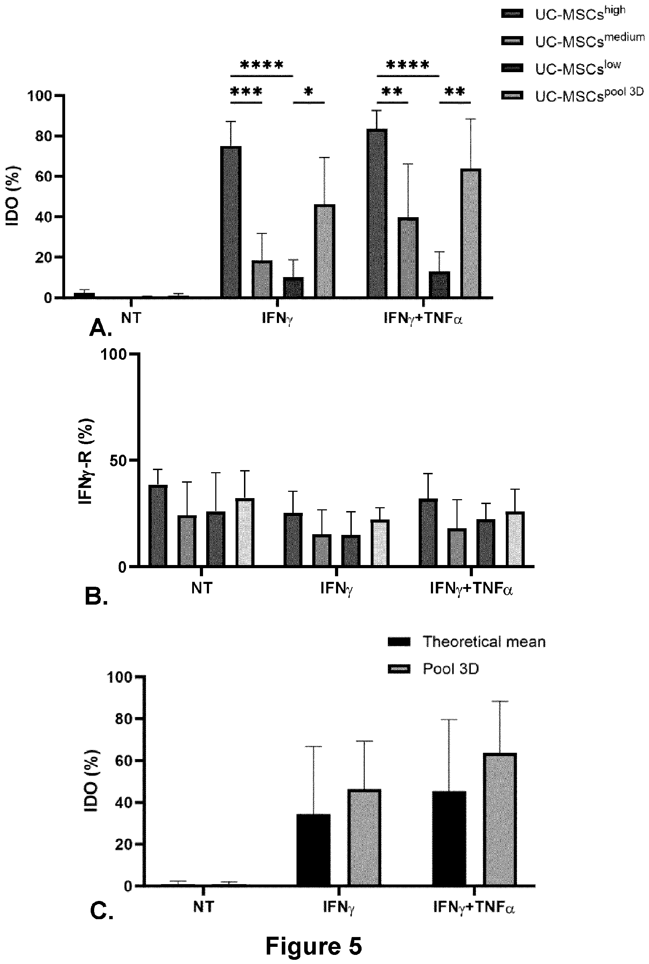

**Figure 5****: Expression of IDO and IFNγ-Receptor in UC-MSCs isolated from individual donors versus pool.** Expression (%) of **A.** IDO (individual donors versus pool) **B.** IFNγ-Receptor and **C.** IDO (theoretical mean of individual donors versus pool) at basal state or under pro-inflammatory conditions (treatment with IFNγ or IFNγ+TNFα).

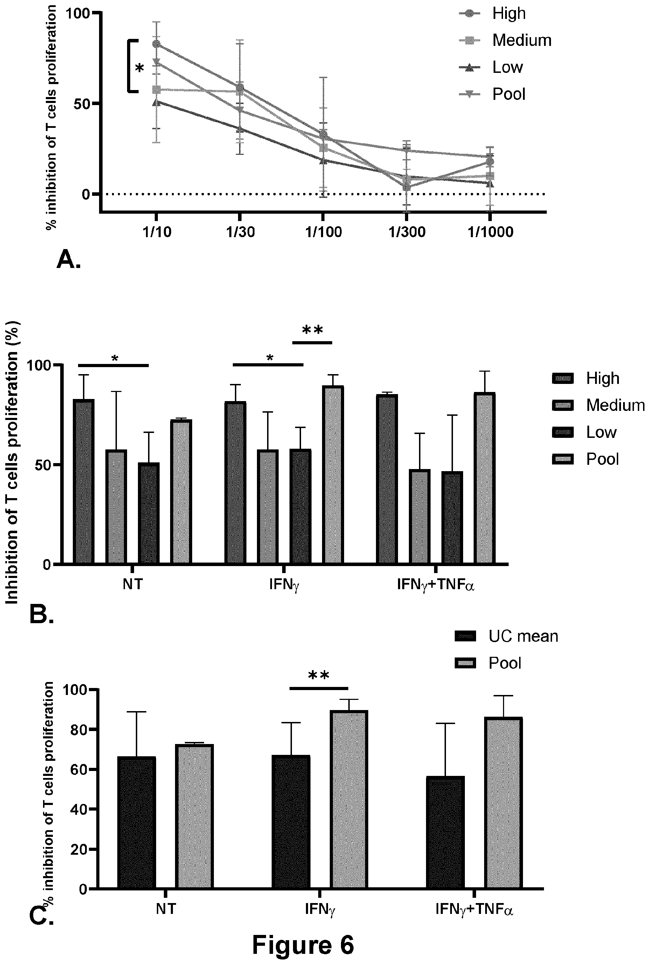

**Figure 6****: suppression of T-cells expansion *in vitro* with a ratio -MSCs:PBMC. A.** untreated MSCs with a decreased UC-MSCs: PBMC ratio. **B.** MSCs at basal state or under pro-inflammatory conditions with a UC-MSCs:PBMC =1:10 ratio. **C.** theoretical average of the three donors versus pool.

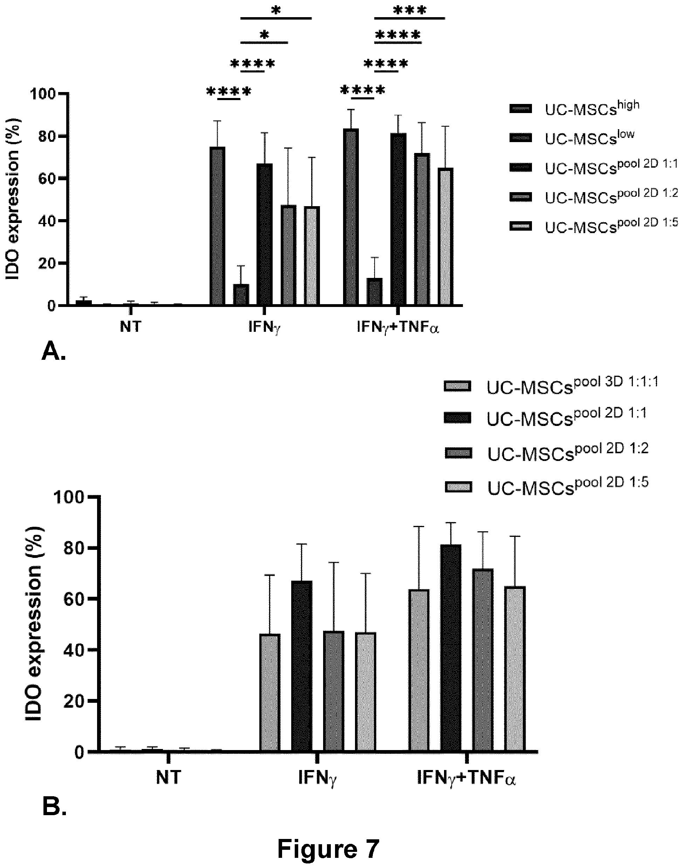

**Figure 7****: Expression of IDO in UC-MSCs isolated from high and low donors versus pool of 2 or 3 donors.** Expression (%) of **A.** IDO of cells from individual donors and 2 donors (2D) pools at different ratio **B.** IDO of cells from 2D and 3D pools.

### EXAMPLES

### Example 1. Material and methods

### 1. UC-MSCs isolation and expansion

UCs were collected from 10 healthy donors, according to the French regulation. UCs collection, UC-MSCs isolation and expansion were performed as previously described (Mebarki et al, Stem Cell Res Ther. 2021 Nov 13;12(1):571).

### 2. Preparation of UC-MSCs pool

Cells isolated from single donors were thawed between passages 2 and 4 maximum, washed, enumerated then seeded at a density of 1000 cells/cm² for each donor, in 75cm² culture flasks using a GMP-compliant complete culture medium composed of NutriStem^{®} MSC XF Basal Medium (Biological Industries, Ref 05-200-1A) + Nutristem^{®} MSC XF Supplement Mix (Biological Industries, Ref 05-200-1U) + 5% irradiated platelet lysate (PL) MultiPL100'i (Macopharma, Ref BC0190032) + sodium Heparin 2 IU/mL (Panpharma, Ref 5520508). UC-MSCs were harvested using a recombinant trypsin when they reached 80% of confluence. Harvested cells from each single donor were pooled in pools of 2 (2D) or 3 donors (3D) then used for adequate analysis.

### 3. Cell proliferation assessment

UC-MSCs from individual donors and from pools were enumerated after each passage using a manual Malassez counting Chamber. Cells viability was assessed using an automatic cell counter EVE Plus 1 (Ozyme). Time Doubling (TD) and Population Doubling (PD) were determined after each passage according to the formulas (T × log(2))/(log Y - log X) and log(Y/X)/log(2) respectively (X: number of cells originally seeded, Y: final number of cells, T: duration of culture in hours).

### 4. Priming with pro-inflammatory cytokines

In order to assess the immunomodulatory properties of UC-MSCs, an inflammatory environment was created *in vitro* to mimic that seen in patients. UC-MSCs were treated with the pro-inflammatory cytokines IFNγ 10 ng/mL and IFNγ+TNFα 10+10 ng/mL, during 48h. A not-treated (NT) condition was used to assess UC-MSCs at a basal state. After conditioning with cytokines, cells were harvested using a recombinant trypsin EDTA solution (Biological Industries, Ref 03-079-1B), washed and suspended in the adequate medium depending on the analysis.

### 5. UC-MSCs phenotype

UC-MSCs phenotype was assessed for each single donor then for the pool, at basal state (NT) and after pro-inflammatory priming, as previously described (Mebarki et al., Stem Cell Res Ther. 2021 Nov 13;12(1):571). Briefly, cells were suspended in 100 µL PBS/albumin 1% and stained concomitantly with several antibodies divided in two panels (additional file 1), for 15 min at 4°C, protected from the light. Cells were washed in 1 ml PBS/Albumin 1%, centrifuged at 1500 RPM for 5min and suspended in 300 µL PBS/albumin 1%. Negative controls were non-stained cells or Fluorescence Minus One for CD31, CD14 and CD45 antibodies. The acquisitions were performed with Attune NxT^{™} Thermofisher^{®} Flow Cytometer and analyzes were performed using Attune NxT software.

### 6. Activation markers expression

The expression of activation markers was evaluated on UC-MSCs of single donors then on pooled cells, at basal state and after priming. The expression of Indoleamine 2,3-dioxygenase (IDO), Inter Cellular Adhesion Molecule-1 (ICAM-1/CD54), Programmed Death-Ligand 1 (PD-L1/CD274), Vascular Cell Adhesion Molecule 1 (VCAM-1/CD106), CD200, INFγ-Receptor (INFy-R/CD119) and TNFα-Receptor II (TNF-RII/CD120b) were assessed according to the protocol previously described (additional file 2) (10). Acquisitions were performed using Attune NxT^{™} Thermofisher^{®} Flow Cytometer and analyses using Attune NxT^{™} software.

### 7. Mixed Lymphocyte Reaction (MLR)

MLR potency assay was performed according to Nicotra et al. Stem Cell Res Ther. 2020 Oct 1;11(1):426. MLR assay was performed on UC-MSCs both in a resting state (NT) and after pro-inflammatory conditioning during 48 hours. Peripheral blood mononuclear cells (PBMC) pooled from 10 healthy donors were co-cultured with UC-MSCs from each donor or with UC-MSCs pooled from 3 donors. The ratio of UC-MSCs:PBMC 0:1 (control), 1:10, 1:30, 1:100, 1:300 and 1:1000 were used.

### 8. Statistical analysis

Statistical analyses were performed using GraphPad PRISM^{®} 10.1.2 software with appropriate tests. Descriptive data are expressed as median [min - max] and all other values are expressed as mean ± standard deviation. A minimum of 95% confidence interval was established for significance. A p-value < 0.05 was considered statistically significant. Kruskal-Wallis test was used to compare 10 donors and Tukey's test was used for multiple comparison.

### Example 2. Results

### 1. Characteristics of collected UCs

UCs were collected from 10 healthy mothers, aged from 27 to 37 years (Table 1). The median full term was 40.1 W [39 - 41 W], the delivery was 9/10 vaginal and 1/10 caesarian and the sex ratio of newborns was 4 females and 6 males (F/M = 0.67), with median weight = 3480 g [2910 - 4200 g]. UCs weight was 22.0 g [15 - 43 g]; nine of them were manipulated within 1 day and one within 3 days.

Isolated cells presented morphological and phenotypic properties of UC-MSCs as previously described (data not shown) (Mebarki et al., Stem Cell Res Ther. 2021 Nov 13;12(1):571).

**Table 1. Characteristics of collected UCs (n = 10 donors). UC 5A: UC used in the clinical trial NCT04333368. NA: not available, g: grams, d: days, w: weeks, y: years.**

| UC | Donor age (y) | Full term (w) | Delivery | Child gender | Child weight (g) | UC weight (g) | Procurement (d) |
|---|---|---|---|---|---|---|---|
| UC 179 | 27 | 40,3 | Vaginal | F | 4200 | 24 | 1 |
| UC 180 | 33 | 39,6 | Cesarean | F | NA | 16 | 3 |
| UC 184 | 37 | 39,4 | Vaginal | F | NA | 25 | 1 |
| UC 185 | 34 | 40,7 | Vaginal | M | NA | 15 | 1 |
| UC 186 | 37 | 39 | Vaginal | M | 3930 | 43 | 1 |
| UC 187 | 36 | 40,7 | Vaginal | M | 3480 | 36 | 1 |
| UC 189 | 35 | 41 | Vaginal | M | 2910 | 16 | 1 |
| UC 190 | 27 | 41 | Vaginal | M | 3420 | 15 | 1 |
| UC 191 | 30 | 39,8 | Vaginal | M | 3290 | 26 | 1 |
| UC 5A | 29 | 39 | Vaginal | F | 3620 | 20 | 1 |

### 2. UC-MSCs immunomodulatory functions are donor-dependent

Immunomodulatory properties of UC-MSCs derived from each donor were analyzed. In order to limit the use of the master and work cell stocks (MCS and WCS), descriptive analysis was performed on n=1/donor. First, the expression of IDO was assessed, at basal state and in pro-inflammatory conditions (Figure 1A). IDO was lowly expressed in non-treated UC-MSCs (0.92% [0.04 - 18.68]) and was induced after priming with IFNγ (69.28% [1.28 - 87.38], p=0.0022) and IFNγ+TNFα (67.22% [2.01 - 92.48], p=0.0017). Even after pro-inflammatory priming, an important heterogeneity was observed between donors, with IDO expression > 60% for seven donors and ≤ 30% for three donors, including one donor with IDO ≤ 2%. To confirm this variability and determine whether all collected UCs are useful in clinic, a MLR potency assay previously validated as a GMP-compliant Quality Control for UC-MSCs-based ATMPs was performed (Nicotra et al., Stem Cell Res Ther. 2020 Oct 1;11(1):426). To mimic the pharmaceutical manufacturing of an ATMP, MLR assay was performed on UC-MSCs at basal state (non-treated condition). The median inhibition of T cells proliferation with ratio 1:30 was 53.1% [35.5 - 96.1 %] and was used as a specification to validate the conformity of MLR assay (Figure 1B). Based on this parameter, 5 UCs were compliant (> 53.1%) including UC185 (96.1%), UC180 (94.7%), UC190 (76.7%), UC191 (59.5%) and UC189 (55.0%) and 5 were out-of-specifications (OOS) with UC186 (51.1%), UC5A (49.3%), UC184 (42.1%), UC179 (36.5%) and UC187 (35.5%). Then, the interquartile range was used to distinguish 2 profiles within compliant products: UC185 and UC180 displayed a high capacity (> 81.2%) to inhibited T cell proliferation whereas UC190, UC191 and UC189 presented medium inhibition function. All OOS products were classified as UCs with low immunomodulatory potential.

Based on these data, the MLR functional assay was used to distinguish 3 profiles of UC-MSCs immunosuppressive functions: high, medium and low.

### 3. Constitution of UC-MSCs pool

To better assess the donor-dependent variability of UC-MSCs biological properties, analysis on UC-MSCs isolated from UCs of each profile was performed: high (UC180), medium (UC190) and low (UC184) that we named UC-MSCs^{high}, UC-MSCs^{medium} and UC-MSCs^{low} respectively. Then, properties of UC-MSCs from individual donors were compared to a pool of UC-MSCs from these 3 donors (UC-MSCs^{pool}). UC-MSCs^{pool} consisted of UC-MSCs^{high}, UC-MSCs^{medium} and UC-MSCs^{low} at ratio 1:1:1 (Figure 2A).

Cells morphology was not modified in the pool. UC-MSCs from individual donors of each profile (UC-MSCshigh, UC-MSCsmedium and UC-MSCslow) and from the pool expressed mesenchymal surface markers (CD90, CD73, CD105, CD29) and did not express hematological neither endothelial markers (CD45, CD14, CD31) before and after the pro-inflammatory treatment (not shown).

As the manufacturing of cell based-therapies is time and cost consuming, MSCs proliferative potential *in vitro* is a critical parameter for donor selection. the theoretical mean TD of UC-MSCs from each donor was similar to the TD of pooled UC-MSCs in non-treated and treated conditions (Figure 2B).

### 4. Pooling UC-MSCs allows to standardize the expression of adhesion surface markers

Despite the growing interest of MSCs paracrine properties and the use of their extracellular vesicles, cell-to-cell interaction is still a key parameter to guarantee a full efficacy of MSCs immunosuppressive functions (21,22). Thus, the expression of several markers described to be involved in MSCs cell-to-cell direct mechanisms was assessed. At basal state (non-treated) UC-MSCs from each donor and from the pool expressed several adhesion molecules including CD44, activated-leukocyte cell adhesion Molecule (ALCAM/CD166), the intercellular adhesion molecule-1 (ICAM-1/CD54), melanoma cell adhesion molecule (MCAM/CD146) and CD200.

The expression of CD44 was similar between individual donors and compared to pooled UC-MSCs, in non-treated condition as well as after pro-inflammatory treatment (Figure 3A). ALCAM/CD166 showed donor-dependent heterogeneity in non-treated UC-MSCs with increased expression in UC-MSCs^{medium} (83.1±18.6%) compared to UC-MSCs^{high} (32.1±30.8%, p=0.0253) and UC-MSCs^{low} (43.9±8.5%, p>005) (Figure 3B). Pooling cells allowed increasing the expression of ALCAM/CD166 (81.1±7.1%) that became similar with UC-MSCs^{medium} and higher than UC-MSCs^{high} (p=0.0186) and UC-MSCs^{low} (p>0.05). In pro-inflammatory conditions, the expression of ALCAM/CD166 was still donor-dependent and was increased in pooled UC-MSCs (Figure 3B). Similar results were observed with MCAM/CD146, with increased expression after pooling UC-MSCs at basal state as well as in pro-inflammatory conditions (Figure 3C). The expression of ICAM-1/CD54 at basal state was higher in UC-MSCs^{high} (85.1±13.5%) than in UC-MSCs^{medium} (28.4±17.5%, p=0.0004) and UC-MSCs^{low} (59.3±44.7%, p>0.05). Pooling cells did not allow to enhance the lowest expression of ICAM-1/CD54, whereas pro-inflammatory treatment increased the expression to > 95% in individual donors and the pool (Figure 3D). The expression of CD200 was also higher in UC-MSC^{high} at basal state and was still similar after pro-inflammatry treatment. Pooling UC-MSCs increased CD200 expression comparing to UC-MSCs^{medium} and UC-MSCs^{low} but was not significant (Figure 3E).

The vascular cell adhesion molecule-1 (VCAM-1/CD106) and programmed cell death protein 1 (PD-L1) were not expressed at basal state but were stimulated after pro-inflammatory treatment (Figure 2F-G). After treatment with IFNγ+TNFα, VCAM-1/CD106 was highly expressed in UC-MSCs^{medium} whereas PD-L1 increased in UC-MSCs^{high} and UC-MSCs^{low}. UC-MSCs^{pool} allowed to increase the lowest expression of VCAM-1/CD106 and PD-L1 but was still not significant comparing to each individual donor.

Taken together, these results highlight the existence of an important donor-to-donor variability of the expression of adhesion surface markers, which tends to be standardized by pooling UC-MSCs.

### 5. The low immunogenicity of UC-MSCs is still not modified in the pooled product

Neither cells of individual donors or in the pool did express HLA-DR at basal state. After IFNγ priming, HLA-DR expression was strongly increased in UC-MSCs^{high} comparing to UC-MSCs^{medium} and UC-MSCs^{low} (26.3±11.1% vs 2.4±1.7% vs 1.9±1.6%, p<0.0001) (Figure 4A). Similar results were observed with IFNγ+TNFα (13.9±5.0% vs 0.8±0.7% vs 1.0±0.9%, p<0.01) (Figure 4A). Interestingly, after IFNγ conditioning HLA-DR was higher in UC-MSCs^{pool} than in UC-MSCs^{medium} and UC-MSCs^{low} (12.4±4.1% vs 2.4±1.7% vs 1.9±1.6%, p<0.05), but was still lower than in UC-MSCs^{high} (12.4±4.1% vs 26.3±11.1%, p<0.01) (Figure 4A). The expression of HLA-DR by UC-MSCs^{pool} was similar with the theoretical mean of individual donors, after treatment with IFNγ (12.4±4.1 vs 10.18±13.3%) and IFNγ+TNFα (6.0±5.1% vs 5.2±7.0%) (Figure 4B). In parallel, the expression of co-stimulatory molecules CD40 and CD86 was also heterogeneous between donors but presented similar profile between UC-MSCs^{pool} and UC-MSCs^{high} (Figure 4C). These results demonstrated that allogeneic UC-MSCs pooled from several donors did not induce a cumulative immunogenic effect, which is encouraging for the clinical use of pooled UC-MSCs-based ATMPs as an allogeneic product.

### 6. IFNy-stimulated and pooled UC-MSCs enhance the lowest expression of IDO

IDO was not expressed at basal state but was enhanced in pro-inflammatory conditions, with donor-dependent variability (Figure 5A). After treatment with IFNγ and IFNγ+TNFα, IDO expression was significantly higher in UC-MSCs^{high} than in UC-MSCs^{medium} and UC-MSCs^{low} (IFNγ: 75.1±12.1% vs 18.5±13.4% vs 10.1±8.7%; p=0.0003 and p<0.0001 respectively; IFNγ+TNFα: 83.5±9.0% vs 39.8±26.4% vs 13.0±9.7%; p=0.0048 and p<0.0001 respectively; Figure 5A). UC-MSCs^{pool} raised significantly the IDO expression comparing with UC-MSCs^{low} (IFNγ: 46.4±23.0% vs 10.1±8.7%, p=0.0217; IFNγ+TNFα: 63.8±24.6% vs 13.0±9.7; p=0.001; Figure 5A). Interestingly, these differences were observed despite the uniform expression of IFNγ-Receptor (IFNγ-R, CD119) on each donor, as well as on pooled UC-MSCs (Figure 5B). Moreover, the IDO expression by UC-MSCs^{pool} was similar to the theoretical mean of IDO expression by the three donors, after treatment with IFNγ (46.4±23.0 vs 34.6±32.3%) and IFNγ+TNFα (63.8±24.6% vs 45.4±34.2%) suggesting that pooling UC-MSCs allows to average IDO expression thus, reducing the inter-donor variability (Figure 5C).

### 7. Pooling UC-MSCs increase their potential to inhibit T cell proliferation

In previous work, it was demonstrated through a MLR potency assay (Nicotra et al., Stem Cell Res Ther. 2020 Oct 1;11(1):426) the capacity of UC-MSCs to inhibit T-cell proliferation in a IFNγ-independent manner and the increase of this inhibition after pro-inflammatory stimulation (Mebarki et al, Stem Cell Res Ther. 2021 Nov 13;12(1):571.). In this study, it was confirmed that at basal state (untreated), UC-MSCs from each single donor as well as from the pool, are able to suppress T-cells expansion *in vitro* in a dose-dependent manner (Figure 6A). This suppression was > 50% in each single donor and in the pool when UC-MSCs:PBMC ratio was 1:10 and decreased significantly when the ratio UC-MSCs:PBMC was 1:100 for UC-MSCs^{pool} and 1:300 for UC-MSCs^{high} and UC-MSCs^{low}. However, this activity was donor-dependent given that with the ratio UC-MSCs:PBMC = 1:10, the suppression of T cells proliferation was more important in UC-MSCs^{high} than in UC-MSCs^{low} (82.9±12.1% vs 51.1±15.1%, p=0.0278) before treatment as well as after priming with IFNγ (81.9±8.2% vs 58.0±10.8%, p=0.0204) and, tends to be higher with IFNγ+TNFα (85.3±1.2% vs 46.8±28.0%, p>0.05) (Figure 5B). Interestingly, UC-MSCs^{pool} approximates the potential of UC-MSCs^{high}. After IFNγ treatment, the inhibition of T-cells expansion by UC-MSCs^{pool} was higher than UC-MSCs^{low} (89.7±5.4% vs 58.0±10.8%, p=0.0062, Figure 5B) and than the theoretical average of the three donors (89.7±5.4% vs 67.1±16.4%, p=0.0053, Figure 5C).

Taken together, these results suggest that pro-inflammatory stimulation did not decrease the donor-dependent variability whereas the use of pooled UC-MSCs improves low immunomodulatory functions.

### 8. Pooling UC-MSCs from high and low donors with decreased ratio of UC-MSCs^{hi9h} is sufficient to increase the lowest immunoregulatory functions

In order to optimize the constitution of pools, the immunomodulatory properties of UC-MSCs pooled from two donors, high and low, named UC-MSCs^{pool 2D} was assessed. Regarding the value of UC-MSCs^{high} dose de-escalation of these cells in this pool was performed. Thus, UC-MSCs^{pool 2D} consisted of UC-MSCs^{high} and UC-MSCs^{low} at ratio 1:1; 1:2 and 1:5 respectively. At basal state, IDO was not expressed (<2%) in UC-MSCs^{pool 2D} regardless of the ratio. After treatment with IFNγ, IDO expression was significantly increased in UC-MSCs^{pool 2D} at ratio 1:1 compared with UC-MSCs^{low} (67.1±14.5% versus 10.1±8.7%, p<0.0001) and was maintained higher at ratio 1:2 (47.5±26.9%; p=0.0113) and 1:5 (47.0±23.1%; p=0.0125) (Figure 7A). Priming with IFNγ+TNFα increased the difference between UC-MSCs^{pool 2D} and UC-MSCs^{low} while IDO expression in UC-MSCs^{pool 2D} reaches those in UC-MSCs^{high} (83.5±9.0%) at ratio 1:1 (81.4±8.5%) and still high at ratio 1:2 (71.9±14.4%) and 1:5 (65.0±19.6%). Importantly, IDO expression in UC-MSCs pooled from two donors even at low ratio, was similar with the three donors pool (Figure 7B).

## Claims

1. A method for grading the immunomodulatory property of human mesenchymal stromal cells (MSCs), comprising
a. Revealing immunomodulatory properties of mesenchymal stromal cells cultured in a resting state and/or primed in pro-inflammatory conditions
b. Obtaining a numerical value associated with the immunomodulatory property of the cells
c. Grading the immunomodulatory property of the population of cells as high, medium or low, wherein a grade "high" or "medium" is assigned when the numerical value associated with the immunomodulatory property of the cells is above a median value, and wherein a grade "low" is assigned when the numerical value associated with the immunomodulatory property of the cells is below the median value, wherein the median value is the median value from a series of numerical values associated with the immunomodulatory properties, measured from multiple populations of mesenchymal stromal cells from multiple donors.

2. The method of claim 1, wherein the grade "high" is assigned when the numerical value associated with the immunomodulatory property of the cells is within the last quartile, and wherein the grade "medium" is assigned when the numerical value associated with the immunomodulatory property of the cells is within the third quartile of the series of numerical values associated with the immunomodulatory properties of multiple populations of mesenchymal stromal cells from multiple donors.

3. The method of claim 1, wherein the grade "high" is assigned when the numerical value associated with the immunomodulatory property of the cells is above the sum of median and interquartile, and wherein the grade "medium" is assigned when the numerical value associated with the immunomodulatory property of the cells is above the median and below sum of median and interquartile of the series of numerical values associated with the immunomodulatory properties of multiple populations of mesenchymal stromal cells from multiple donors.

4. The method of any one of claims 1 to 4, wherein the immunomodulating property of the cells is the ability to inhibit lymphocyte proliferation, as measured by Mixed Lymphocyte Reaction.

5. The method of any one of claims 1 to 5, wherein the median value is calculated for values associated with mesenchymal stromal cells from 10 or more donors.

6. The method of any one of claims 1 to 6, wherein the mesenchymal stromal cells are umbilical cord derived mesenchymal stromal cells.

7. A method for preparing a pool of mesenchymal stromal cells, comprising:
a. Providing mesenchymal stromal cells from at least two donors
b. Testing the mesenchymal stromal cells of each donor, with regards to their immunomodulatory properties
c. Selecting first mesenchymal stromal cells from a first donor, wherein the cells present high immunomodulatory properties
d. Selecting second mesenchymal stromal cells from a second donor, wherein the cells present medium or low immunomodulatory properties
e. Mixing the first and second selected mesenchymal stromal cells to obtain a pool of mesenchymal stromal cells.

8. The method of claim 8, wherein testing and grading of the mesenchymal stromal cells is performed by the method of any of claims 1 to 7.

9. The method of claim 8 or 9, further comprising preparing multiple aliquots of the cells of the pool.

10. The method of any one of claims 7 to 10, further comprising freezing the pool or the cells from individual donors.

11. A pool of mesenchymal stromal cells, wherein the pool contains cells from at least two donors, wherein the cells of one donor are graded as having high immunomodulatory property, and wherein the cells of the other donor(s) are graded as having medium or low immunomodulatory property

12. The pool of claim 12, for use thereof as a medicament.

13. A pool of mesenchymal stromal cells, wherein the pool contains cells from at least two donors, wherein the cells of one donor are graded as having high immunomodulatory property, and wherein the cells of the other donor(s) are graded as having medium or low immunomodulatory property, for use thereof for the treatment of immune or inflammatory diseases.

14. The pool of mesenchymal stromal cells for use according to claim 11, wherein the cells are provided at a dose comprised between 10⁵/kg to 10⁷/kg to the patient, in one or more administrations.
